# EUROPÄISCHE PATENTSCHRIFT

(11) **EP 2 254 856 B1**
(45) Veröffentlichungstag und Bekanntmachung des Hinweises auf die Patenterteilung: **24.08.2016**
(21) Anmeldenummer: 09723845.5
(22) Anmeldetag: 13.02.2009
(51) Int. Cl.: C07C 49/792, A61K 8/31, A61K 8/35, A61Q 13/00, C11B 9/00, C07C 45/68, C11D 3/50

(54) **Verfahren zur Beduftung von Oberflächen durch Strahlung von photolabile Duftspeicherstoffe und Herstellung der Duftspeicherstoffe**
Process for fragrancing of surfaces by irradiation of photolabile fragrance storage substances and preparation of the photolabile fragrance storage substances
Procédé pour la profumation des surfaces par irradiation des ingrédients odoriférants photolabiles et préparation des ingrédients odoriférants

(30) Priorität: 28.03.2008 DE 102008016327
(43) Veröffentlichungstag der Anmeldung: 01.12.2010
(73) Patentinhaber: Henkel AG & Co. KGaA, 40589 Düsseldorf (DE)
(72) Erfinder: HUCHEL, Ursula, 50670 Köln (DE); KROPF, Christian, 40724 Hilden (DE); GRIESBECK, Axel, 50937 Köln (DE); HINZE, Olga, 50679 Köln (DE); PEREZ-RUIZ, Raoul, 46120 Alboraya Valencia (ES)
(86) Internationale Anmeldenummer: PCT/EP2009/051674
(87) Internationale Veröffentlichungsnummer: WO 2009/118219

(56) Entgegenhaltungen:
- WO-A1-93/23051
- WO-A2-01/96272
- US-A- 2 686 808
- US-A1- 2004 259 867
- US-A1- 2007 264 217
- K. ALDER ET AL.: BERICHTE DE DEUTSCHEN CHEMISCHEN GESELLSCHAFT, Bd. 76, Nr. 3, 1943, Seiten 183-205, XP002527055
- JOURNAL OF ORGANIC CHEMISTRY, Bd. 36, Nr. 13, 1971, Seiten 1838-1840, XP002527056
- H.C. BROWN ET AL.: JOURNAL OF THE AMERICAN CHEMICAL SOCIETY, Bd. 90, Nr. 22, 1968, Seiten 6218-6219, XP002527057
- XIAO ZHUANQUAN ET AL: "Synthesis of endo-.alpha.-isocamphanyl ketones and .beta.-isocamphanyl alcohols" CHEMISTRY AND INDUSTRY OF FOREST PRODUCTS / LIN CHAN HUA XUE YU GONG YE, CHINESE ELECTRONIC PERIODICAL SERVICES, CHINA, Bd. 16, Nr. 3, 1. Januar 1996 (1996-01-01), Seiten 19-23, XP008106157 ISSN: 0253-2417
- DIAZ-MARRERO A R ET AL: "Caminatal, an aldehyde sesterterpene with a novel carbon skeleton from the Antarctic sponge Suberites caminatus" TETRAHEDRON LETTERS, ELSEVIER, AMSTERDAM, Bd. 44, Nr. 31, 28. Juli 2003 (2003-07-28), Seiten 5939-5942, XP004435111 ISSN: 0040-4039
- IWASAWA, NOBUHARU ET AL: "Synthesis of Medium-Sized Bicyclic Compounds by Intramolecular Cyclization of Cyclic .beta.-Keto Radicals Generated from Cyclopropanols Using Manganese(III) Tris(pyridine-2-carboxylate) and Its Application to Total Synthesis of 10-Isothiocyanatoguaia-6-ene" BULLETIN OF THE CHEMICAL SOCIETY OF JAPAN, Bd. 72, Nr. 1, 1999, Seiten 85-97, XP002527309 ISSN: 0009-2673
- QUINKERT, G. ET AL: "Ketenbildung Durch Intramolekulare Disproportionierung Photochemisch Erzeugter Alkyl/Acyl-Radikalpaare Aus Cyclischen Ketonen" TETRAHEDRON LETTERS, Nr. 6, 1962, Seiten 221-225, XP002527310 ISSN: 0040-4039
- HWU, JIH RU ET AL: "The trimethylsilyl cationic species as a bulky proton. Application to chemoselective dioxolanation" JOURNAL OF ORGANIC CHEMISTRY, Bd. 50, Nr. 20, 1985, Seiten 3946-3948, XP002527353 ISSN: 022-3263
- DATABASE CAPLUS [Online] CHEMICAL ABSTRACTS SERVICE, COLUMBUS, OHIO, US; CROFT K D ET AL: "THE CHEMISTRY OF EREMOPHILA SPP. XXI. STRUCTURAL STUDY OF A NEW EREMANE DITERPENE" XP002527604 Database accession no. 1984:451700 -& AUSTRALIAN JOURNAL OF CHEMISTRY, CSIRO, AU, Bd. 37, Nr. 4, 1. April 1984 (1984-04-01), Seiten 785-793, XP008105986 ISSN: 0004-9425

## Beschreibung

Textil- und Oberflächenbehandlungsmittel bzw. kosmetische Mittel enthalten zumeist Duftstoffe, die den Mitteln einen angenehmen und frischen Geruch verleihen. Die Duftstoffe, die sowohl synthetischer als auch natürlicher Art sein können, maskieren dabei zumeist die Eigenduftnote der anderen Inhaltstoffe, so dass beim Verbraucher ein positiver Geruchseindruck entsteht. Im Bereich Waschmittel sind Duftstoffe besonders wichtige Bestandteile der Zusammensetzung, da die Wäsche sowohl im feuchten als auch im trockenen Zustand einen angenehmen und frischen Duft aufweisen soll. Ein grundsätzliches Problem bei der Verwendung von Duftstoffen besteht hierbei darin, dass es sich bei diesen naturgemäß um flüchtige Substanzen handelt, ansonsten könnte kein Dufteffekt erzielt werden. Man steht daher bei dem Einsatz von Duftstoffen in Wasch- und Reinigungsmitteln ebenso wie bei dem Einsatz in kosmetischen Mitteln vor dem Problem, dass es sich bei den Duftstoffen naturgemäß um flüchtige Verbindungen handelt, man aber andererseits einen langanhaltenden und möglichst gleichbleibenden Dufteffekt bewirken möchte. Hinzu kommt des weiteren, dass sich der Dufteindruck eines Parfums im Laufe der Zeit verändert, weil die Riechstoffe, die die frischen und leichten Noten des Parfüms darstellen durch ihren hohen Dampfdruck schneller abdampfen als die Duftstoffe, die die Herz- und Basisnoten darstellen.

Ein Ansatz zur Lösung dieses Problems besteht darin, Duftstoffe auf Trägermaterialien aufzubringen und die bedufteten Träger zu beschichten, oder Duftstoffe zu verkapseln oder in Verbindungen einzulagern (beispielsweise Cyclodextrin-Parfüm-Komplexe). Des Weiteren existiert die Möglichkeit, die Duftstoffe chemisch an Trägermedien zu binden, wobei die chemische Bindung langsam gespalten und der Duftstoff hierdurch freigesetzt wird. Eine solche Träger-gebundene Vorform eines Duftstoffes wird auch als "Pro-Fragrance" oder Duftspeicherstoff bezeichnet.

In diesem Zusammenhang offenbart die internationale Patentanmeldung WO2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird.

Der beschriebene Prozess muss dabei für eine wirksame Freisetzung des Duftstoffs die Anwesenheit von Sauerstoff und Wasser tolerieren.

In diesem Zusammenhang offenbart das US-Patent 6,949,680 und WO0196272 die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird. Dabei werden die genannten photolabilen Phenyl- oder Pyridylketone als Duftspeicherstoffe in einem aufwendigen, vielstufigen Syntheseverfahren unter Einsatz von Schutzgruppenoperationen hergestellt, wobei die Synthese für jeden einzelnen Aktivstoff individuell angepasst werden muss. Auch offenbart das US-Patent 6,949,680 nicht die photoinduzierte Freisetzung von cyclischen Alkenen mit semicyclischer Doppelbindung als Aktivstoffe oder die Herstellung oder Verwendung der entsprechenden Duftspeicherstoffe. Cyclische Alkene mit semicyclischer Doppelbindung, bei denen es sich beispielsweise um die olfaktorisch bedeutende Klasse der cyclischen Terpene mit semicyclischer Doppelbindung handeln kann, stellen eine wichtige Klasse von Geruchsstoffen dar. Diese können beispielsweise dazu beitragen, Textil- und Oberflächenbehandlungsmittel oder kosmetischen Mitteln einen angenehmen und frischen Geruch zu verleihen. Die genannten Alkene zeichnen sich in der Regel durch ihren hohen Dampfdruck aus und sind aufgrund ihres niedrigen Funktionalisierungsgrads chemisch nur schwer an herkömmliche Trägermedien zu binden. Auch sind keine photoaktivierbaren Substanzen bekannt, welche die genannten Alkene über einen längeren Zeitraum hinweg freisetzen können.

Ziel der vorliegenden Erfindung war daher die Bereitstellung photoaktivierbarer Substanzen als Duftspeicherstoffe, welche die verzögerte Freisetzung von cyclischen Alkenen mit semicyclischer Doppelbindung, insbesondere von cyclischen Terpenen oder cyclischen Terpenoiden mit semicyclischer Doppelbindung erlauben. Ein weiteres Ziel der Erfindung war die Bereitstellung eines einfachen, kostengünstigen und konvergenten Syntheseverfahrens zur Herstellung der genannten Duftspeicherstoffe.

Überraschenderweise wurde nun gefunden, dass bestimmte cyclische Phenylketone als photoaktivierbare Duftspeicherstoffe die verzögerte Freisetzung von cyclischen Alkenen mit semicyclischer Doppelbindung erlauben. Offenbart ist daher ein Keton der allgemeinen Formel (I), 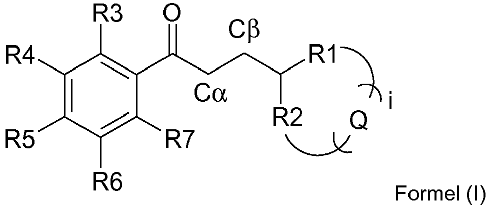 wobei
i =1 oder 2 ist,
R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen und R1 und R2 unabhängig voneinander für ein sekundäres, tertiäres oder quartäres C-Atom stehen und Q für mindestens eine R1 und R2 verbrückende zweiwertige substituierte oder unsubstituierte Gruppe mit 1 bis 10 Kohlenstoffatomen steht.

Unter dem Begriff sekundäres C-Atom ist im Sinne der Erfindung ein Kohlenstoffatom zu verstehen, dass mit zwei weiteren Kohlenstoffatomen kovalent verbunden ist. Unter dem Begriff tertiäres C-Atom bzw. quartäres C-Atom ist im Sinne der Erfindung ein C-Atom zu verstehen, dass mit drei bzw. vier weiteren Kohlenstoffatomen kovalent verbunden ist.

Vorzugsweise stehen R1 und R2 unabhängig voneinander für ein sekundäres oder tertiäres Kohlenstoffatom. In einer ganz besonders bevorzugten Ausführungsform steht einer der beiden Reste R1 und R2 für ein sekundäres Kohlenstoffatom, während der jeweils andere Rest für ein tertiäres Kohlenstoffatom steht.

Weiter ist eine Zusammensetzung offenbart, wie insbesondere ein Wasch- oder Reinigungsmittel (vorzugsweise Textil- oder Oberflächenbehandlungsmittel) oder ein kosmetisches Mittel, enthaltend mindestens ein Keton der allgemeinen Formel (I) wie zuvor definiert, wobei das genannte Keton vorzugsweise in Mengen zwischen 0,001 und 5 Gew.-%, vorteilhafterweise zwischen 0,01 und 4 Gew.-%, weiter vorteilhaft zwischen 0,1 und 3 Gew.-%, insbesondere zwischen 0,5 und 2 Gew.-%, jeweils bezogen auf die Gesamtmenge der Zusammensetzung, enthalten ist.

Gegenstand der vorliegenden Erfindung ist ein Verfahren zur lang anhaltenden Beduftung von Oberflächen, dadurch gekennzeichnet, dass ein Keton der allgemeinen Formel (I) wobei durch Spaltung der Cα-Cß-Bindung ein Keton der allgemeinen Formel (II) und eine Verbindung der allgemeinen Formel (III) gebildet wird, wobei es sich bei der Verbindung der allgemeinen Formel (III) um ein cyclisches Terpen oder cyclisches Terpenoid mit einer semicyclischen Doppelbindung handelt, ausgewählt aus der Gruppe bestehend aus ß-Phellandren (p-Mentha-1 (7),2-dien), α-Fenchen (7,7-Dimethyl-2-methylen-bicyclo[2.2.1]heptan), ß-Fenchen (2,2-Dimethyl-5-methylenbicyclo[2.2.1]heptan), Camphen (2,2-Dimethyl-3-methylenbicyclo[2.2.1]heptan), Sabinen (4-Methylen-1-(1-methylethyl)bicyclo-[3.1.0]hexan), Pinocarveol, ß-Pinen (6,6-Dimethyl-2-methylenbicyclo[3.1.1]heptan), Trichodien (1,4-Dimethyl-4-(1-methyl-2-methylencyclopentyl)cyclohexen), ß-Humulen ((1E,SE)-1,4,4 trimethyl-8- methylenecycloundeca-1,5-dien) oder γ-Humulen ((1E,6E)-1,8,8-trimethyl-5- methylenecycloundeca-1 ,6-dien) sowie aus deren Enantiomeren und/oder Diastereomeren, auf die zu beduftende Oberfläche aufgebracht wird und die genannte Oberfläche anschließend einer elektromagnetischen Strahlung umfassend die Wellenlängen von 200 bis 400 nm ausgesetzt wird. Als elektromagnetische Strahlung im Sinne der vorliegenden Erfindung kann vorzugsweise das natürliche Sonnenlicht gelten.

Ebenfalls Gegenstand der vorliegenden Erfindung ist ein Verfahren zur Herstellung eines erfindungsgemäßen Ketons der allgemeinen Formel (I) umfassend folgende Schritte:
a) Hydroborierung einer Verbindung der allgemeinen Formel (III), 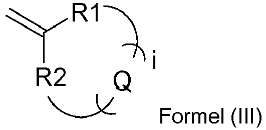 wobei i = 1 oder 2 ist und R1 und R2 unabhängig voneinander für ein sekundäres, tertiäres oder quartäres C-Atom stehen und Q für mindestens eine R1 und R2 verbrückende zweiwertige substituierte oder unsubstituierte Gruppe mit 1 bis 10 Kohlenstoffatomen steht,
b) Umsetzung des in Schritt a) erzeugten Organoboraddukts mit einer Verbindung der allgemeinen Formel (IV), 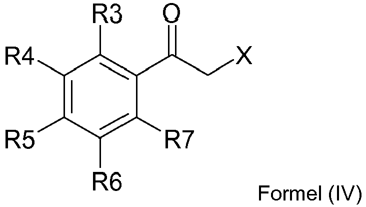 wobei X ein Halogenatom ist und R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

Offenbart ist die Verwendung eines Ketons der allgemeinen Formel (I) als Duftspeicherstoff und die Verwendung eines Ketons der Formel (I) in flüssigen oder festen Waschmitteln, Weichspülern, weichmachenden Waschmitteln, Waschhilfsmitteln oder kosmetischen Mitteln zur Haut- und Haarbehandlung.

Ebenfalls offenbart ist die Verwendung Ketone der Formel (I) insbesondere deren Zusammensetzung wie z.B. Wasch- oder Reinigungsmittel oder kosmetisches Mittel, zur Verbesserung der Duftstoffausbeute, insbesondere auf Textilien.

Offenbart ist ein Keton der allgemeinen Formel (I), in dem i =2 ist und mindestens eine der R1 und R2 verbrückenden, zweiwertigen substituierten oder unsubstituierten Gruppen Q 2 bis 6 C-Atome umfasst.

Weiterhin offenbart ist ein Ketone der allgemeinen Formel (I), in dem i =2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst.

Ebenfalls offebart ist ein Keton der allgemeinen Formel (I), in dem i =2 ist und eine der R1 und R2 verbrückenden, zweiwertigen Gruppen Q verzweigt ist und die kürzeste direkte Verbindung zwischen R1 und R2 aus 2, 3 oder 4 jeweils kovalent miteinander verknüpften Kohlenstoffatomen gebildet wird. Offebart ist insbesondere ein Keton der allgemeinen Formel (I), in dem i =2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst, während die andere R1 und R2 verbrückende, zweiwertige Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Ebenfalls offenbart ist ein Keton der allgemeinen Formel (I), in dem R1 und/oder R2 und/oder mindestens eine R1 und R2 verbrückende zweiwertige Gruppe Q eine Substitution mit mindestens einem Heteroatom ausgewählt aus der Gruppe bestehend aus N, O oder S aufweist. Besonders bevorzugt ist ein Keton der allgemeinen Formel (I), in dem R1 und/oder R2 und/oder mindestens eine R1 und R2 verbrückende zweiwertige Gruppe Q eine Substitution in Form einer -OH, -OR^{a}, -SH, -SR^{b} oder - NR^{c}R^{d}-Gruppe aufweist, wobei R^{a}, R^{b}, R^{c} und R^{d} unabhängig voneinander ausgewählt werden aus der Gruppe bestehend aus Wasserstoff und verzweigten oder unverzweigten, substituierten oder unsubstituierten Alkylgruppen, die 1 bis 10, insbesondere 1 bis 4 und besonders bevorzugt 1, 2 oder 3 Kohlenstoffatome umfassen.

Ebenfalls besonders offenbart ist ein Keton der allgemeinen Formel (I), in dem eine R1 und R2 verbrückende zweiwertige Gruppe Q eine Keton-, Carbonsäure-, Lacton-, Amid-, Ester- und/oder Aldehydstruktur umfasst.

Weiterhin ist ein Keton der allgemeinen Formel (I) offenbart in dem vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, insbesondere -F, -Cl oder -Br, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Offenbarung steht R5 für -Cl, -Br, -NO₂ oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe.

Eine Substitution in para-Position (R5) ist besonders bevorzugt, da die elektronische Struktur des aromatischen Rings hier am effektivsten modifiziert werden kann, wodurch das Absorptionsmaximum von Ketonen der allgemeinen Formel (I) leicht an eine bestimmte Wellenlänge angepasst werden kann.

Ebenfalls bevorzugt ist ein Keton der allgemeinen Formel (I), in dem R3, R4, R5, R6 und R7 für Wasserstoff stehen.

Im Sinne der Erfindung ist ein Keton der allgemeinen Formel (I), aus dem durch Spaltung der Cα-Cβ-Bindung ein Keton der allgemeinen Formel (II) 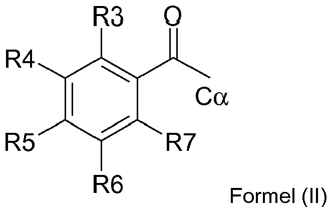 und eine Verbindung der allgemeinen Formel (III) gebildet wird, 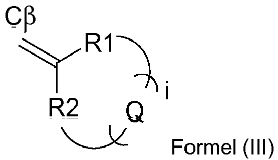 wobei i =1 oder 2 ist, R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen und R1 und R2 unabhängig voneinander für ein sekundäres, tertiäres oder quartäres C-Atom stehen und Q für mindestens eine R1 und R2 verbrückende zweiwertige substituierte oder unsubstituierte Gruppe mit 1 bis 10 Kohlenstoffatomen steht, wobei es sich bei der Verbindung der allgemeinen Formel (III) um ein cyclisches Terpen oder cyclisches Terpenoid mit einer semicyclischen Doppelbindung handelt, ausgewählt aus der Gruppe bestehend aus β-Phellandren (p-Mentha-1 (7),2-dien), α-Fenchen (7,7-Dimethyl-2-methylen-bicyclo[2.2.1]heptan), ß-Fenchen (2,2-Dimethyl-5-methylenbicyclo[2.2.1]heptan), Camphen (2,2-Dimethyl-3-methylenbicyclo[2.2.1]heptan), Sabinen (4-Methylen-1-(1-methylethyl)bicyclo-[3.1.0]hexan), Pinocarveol, β-Pinen (6,6-Dimethyl-2-methylenbicyclo[3.1.1]heptan), Trichodien (1,4-Dimethyl-4-(1-methyl-2-methylencyclopentyl)cyclohexen), ß-Humulen ((1E,5E)-1,4,4 trimethyl-8- methylenecycloundeca-1 ,5-dien) oder γ-Humulen ((1E,6E)-1 ,8,8-trimethyl-5- methylenecycloundeca-1 ,6-dien) sowie aus deren Enantiomeren und/oder Diastereomeren.

Vorzugsweise stehen R1 und R2 in Formel (III) unabhängig voneinander für ein sekundäres oder tertiäres Kohlenstoffatom. Insbesondere steht einer der beiden Reste R1 und R2 für ein sekundäres Kohlenstoffatom, während der jeweils andere Rest für ein tertiäres Kohlenstoffatom steht.

Bevorzugt ist eine Verbindung der allgemeinen Formel (II), in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Erfindung steht R5 für -Cl, -Br, -NO₂ oder eine Alkyl- oder Alkoxygruppe umfassend 1 bis 4 C-Atome.
Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe.

Offenbart ist ein Keton der allgemeinen Formel (I), aus dem durch Spaltung der Cα-Cβ-Bindung eine Verbindung der allgemeinen Formel (III) hervorgeht, in der i =2 ist und mindestens eine der R1 und R2 verbrückenden, zweiwertigen substituierten oder unsubstituierten Gruppen Q 2 bis 6 C-Atome umfasst.

Besonders offenbart ist dabei eine Verbindung der allgemeinen Formel (III), in der i = 2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst.

Ebenfalls offenbart ist eine Verbindung der allgemeinen Formel (III), in der i =2 ist und eine der R1 und R2 verbrückenden, zweiwertigen Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Weiter ist ein Keton der allgemeinen Formel (I) offenbart, aus dem durch Spaltung der Cα-Cβ-Bindung eine Verbindung der allgemeinen Formel (III) hervorgeht, in der i =2 ist und eine der R1 und R2 verbrückenden, zweiwertigen Gruppen Q unverzweigt ist und 2, 3 oder 4 Kohlenstoffatome, insbesondere 3 oder 4 Kohlenstoffatome umfasst, während die andere R1 und R2 verbrückende, zweiwertige Gruppen Q verzweigt ist und die kürzeste direkte Verbindung zwischen R1 und R2 aus 2, 3 oder 4 jeweils kovalent miteinander verknüpften Kohlenstoffatomen gebildet wird.

Ebenfalls offenbart ist eine Verbindung der allgemeinen Formel (III), in der R1 und/oder R2 und/oder mindestens eine R1 und R2 verbrückende zweiwertige Gruppe Q eine Substitution mit mindestens einem Heteroatom ausgewählt aus der Gruppe bestehend aus N, O oder S aufweist. Besonders offenbart ist eine Verbindung der allgemeinen Formel (III), in der R1 und/oder R2 und/oder mindestens eine R1 und R2 verbrückende zweiwertige Gruppe Q eine Substitution in Form einer -OH, -OR^{a}, -SH, -SR^{b} oder -NR^{c}R^{d}-Gruppe aufweist, wobei R^{a}, R^{b}, R^{c} und R^{d} unabhängig voneinander ausgewählt werden aus der Gruppe bestehend aus Wasserstoff und verzweigten oder unverzweigten, substituierten oder unsubstituierten Alkylgruppen, die 1 bis 10 Kohlenstoffatome umfassen.

Ebenfalls besonders offenbart ist eine Verbindung der allgemeinen Formel (III), in der eine R1 und R2 verbrückende zweiwertige Gruppe Q eine Keton-, Carbonsäure-, Lacton-, Amid-, Ester- und/oder Aldehydstruktur umfasst.

Weiterhin offenbart ist eine Verbindung der allgemeinen Formel (III), bei der es sich um ein cyclisches Terpen oder cyclisches Terpenoid mit einer semicyclischen Doppelbindung handelt. Unter dem Begriff der "semicyclischen Doppelbindung" ist im Sinne der Erfindung eine Doppelbindung zwischen zwei Kohlenstoffatomen zu verstehen, in der das eine Kohlenstoffatom der Doppelbindung eine exocyclische Position einnimmt, während das andere Kohlenstoffatom der Doppelbindung Teil eines Ringsystems ist.

Die nachfolgende Zeichnung (B-I) soll den Begriff der semicyclischen Doppelbindung weiter erläutern.

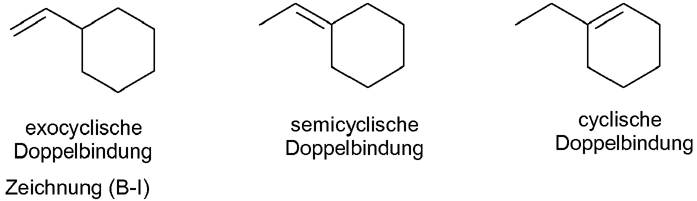

Unter Terpenen sind erfindungsgemäß alle aus Isoprenbasiseinheiten aufgebauten Naturstoffe und Derivate zu verstehen.

Unter Terpenoiden sind erfindungsgemäß alle aus Isoprenbasiseinheiten aufgebauten Naturstoffe und Derivate zu verstehen, die eine hohe strukturelle Verwandtschaft zu den Terpenen aufweisen, sich von diesen aber beispielsweise durch den Verlust oder die Umlagerung eines Fragments, vorzugsweise einer Methylgruppe unterscheiden.

Je nach Zahl der Basiseinheiten teilt man die Terpene in Monoterpene, Sesquiterpene, Diterpene, Sesterpene, Triterpene und Tetraterpene ein.

Gemäß einer bevorzugten Ausführungsform der Offenbarung sind insbesondere monocyclische Monoterpene, bicyclische Monoterpene und cyclische Sesquiterpene. Alle Terpene des erfindungsgemäßen Verfahren umfassen dabei mindestens eine semicyclische Doppelbindung, deren exocyclisches Kohlenstoffatom vor der Spaltung der Cα-Cβ-Bindung in dem erfindungsgemäßen Keton der allgemeinen Formel (I) mit dem Cα-Atom kovalent verknüpft ist, wobei die cyclische Terpene oder cyclische Terpenoide mit semicyclischer Doppelbindung werden aus der Gruppe bestehend aus β-Phellandren (p-Mentha-1(7),2-dien), α-Fenchen (7,7-Dimethyl-2-methylen-bicyclo[2.2.1]heptan), β-Fenchen (2,2-Dimethyl-5-methylenbicyclo[2.2.1]heptan), Camphen (2,2-Dimethyl-3-methylenbicyclo[2.2.1]heptan), Sabinen (4-Methylen-1-(1-methylethyl)bicyclo-[3.1.0]hexan), Pinocarveol, β-Pinen (6,6-Dimethyl-2-methylenbicyclo[3.1.1]heptan), Trichodien (1,4-Dimethyl-4-(1-methyl-2-methylencyclopentyl)cyclohexen), β-Humulen (1E,5E)-1,4,4 trimethyl-8-methylenecycloundeca-1,5-dien oder oder γ-Humulen (1E,6E)-1,8,8-trimethyl-5-methylenecycloundeca-1,6-dien ausgewählt. Ebenfalls besonders bevorzugt sind alle möglichen Enantiomere oder Diastereomere der genannten cyclischen Terpene oder cyclischen Terpenoide mit semicyclischer Doppelbindung.

Die genannten cyclischen Terpene oder cyclischen Terpenoide mit semicyclischer Doppelbindung zeichnen sich in der Regel durch eine hohe Isomerisierungstendenz aus. Bei langer Sonnenlichtexposition kann es zu Gerüstumlagerungen (z.B. durch Wagner-Meerwein-Umlagerungen) oder zur Bildung von Doppelbindungsisomeren kommen. Dies ist unerwünscht, da die genannten Isomere sich oft deutlich in ihrem Geruchseindruck von den ursprünglich vorhandenen cyclischen Terpenen oder cyclischen Terpenoiden mit semicyclischer Doppelbindung unterscheiden. Unter Umständen kann so der Dufteindruck einer Mehrkomponenten-Parfümölmischung entscheidend verändert werden. Ein Vorteil der vorliegenden Erfindung ist daher die Tatsache, dass die erfindungsgemäßen Terpene oder Terpenoide erst direkt bei der Anwendung durch Sonnenlichtexposition freigesetzt werden, wodurch eine Duftveränderung durch vorhergehende Isomerisierungsreaktionen nahezu ausgeschlossen werden kann.

Gegenstand der vorliegenden Offenbarung ist ebenfalls eine Zusammensetzung, enthaltend mindestens eine Verbindung der allgemeinen Formel (la), 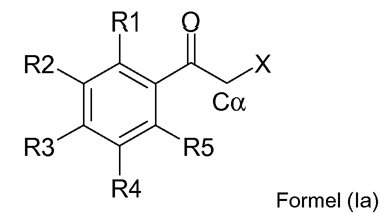 wobei
R1, R2, R3, R4 und R5 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen,
und X nach Spaltung der Cα-X-Bindung für eine zyklische Verbindung, enthaltend mindestens eine semicyclische Doppelbindung steht, wobei das exocyclische Kohlenstoffatom der semicyclische Doppelbindung vor der Spaltung der Cα-X-Bindung kovalent mit dem Cα-Atom verknüpft ist.

Insbesondere handelt es sich bei dem Rest X nach Spaltung der Cα-X-Bindung um ein cyclisches Terpen mit semicyclischer Doppelbindung, ausgewählt aus der Gruppe bestehend aus β-Phellandren (p-Mentha-1(7),2-dien), α-Fenchen (7,7-Dimethyl-2-methylen-bicyclo[2.2.1]heptan), β-Fenchen (2,2-Dimethyl-5-methylenbicyclo-[2.2.1]heptan), Camphen (2,2-Dimethyl-3-methylenbicyclo[2.2.1]heptan), Sabinen (4-Methylen-1-(1-methylethyl)bicyclo-[3.1.0]hexan), Pinocarveol, β-Pinen (6,6-Dimethyl-2-methylenbicyclo-[3.1.1]heptan), Trichodien (1,4-Dimethyl-4-(1-methyl-2-methylencyclopentyl)cyclohexen), β-Humulen (1E,5E)-1,4,4 trimethyl-8-methylenecycloundeca-1,5-dien oder γ-Humulen (1E,6E)-1,8,8-trimethyl-5-methylenecycloundeca-1,6-dien. Ebenfalls besonders bevorzugt sind alle möglichen Enantiomere oder Diastereomere der genannten cyclischen Terpene oder cyclischen Terpenoide mit semicyclischer Doppelbindung.

Bevorzugt ist eine Verbindung der allgemeinen Formel (la), in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO2, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Erfindung steht R5 für -Cl, -Br, -NO2 oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe.

Vorzugsweise enthält die Zusammensetzung, wie z.B. Wasch- oder Reinigungsmittel oder kosmetisches Mittel, mindestens einen weiteren Duftstoff. Die bevorzugt eingesetzten Duftstoffe bzw. Parfümöle sind keinerlei Beschränkungen unterworfen. So können vorzugsweise als Duftstoffe synthetische oder natürliche Riechstoffverbindungen vom Typ der Ester, Ether, Aldehyde (Duftaldehyde), Ketone (Duftketone), Alkohole, Kohlenwasserstoffe, Säuren, Kohlensäureester, aromatischen Kohlenwasserstoffe, aliphatischen Kohlenwasserstoffe, gesättigten und / oder ungesättigten Kohlenwasserstoffe und Mischungen daraus verwendet werden.

Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschten Duft oder ein Frischeempfinden verleihen können. Es können auch Gemische unterschiedlicher Ketone eingesetzt werden. Einsetzbare Ketone sind z.B. Alpha Damascon, Delta Damascon, Iso Damascon, Carvon, Gamma-Methyl-ionon, Iso-E-Super, 2,4,4,7-Tetramethyl-oct-6-en-3-on, Benzylaceton, Beta Damascon, Damascenon, Methyldihydrojasmonat, Methyl-cedrylon, Hedion und Gemischen davon. Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Lilial usw. Die Duftaldehyde und Duftketone eine aliphatische, cycloaliphatische, aromatische, ethylenisch ungesättigte Struktur oder eine Kombination dieser Strukturen aufweisen. Es können ferner weitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander Published 1960 and 1969 respectively, Reprinted 2000 ISBN: Aroma Chemicals Vol. 1: 0-931710-37-5, Aroma Chemicals Vol. 2: 0-931710-38-3, verwiesen. Geeignete Duftstoffe vom Typ der Ester sind beispielsweise Benzylacetat, Phenoxyethylisobutyrat, p-tert.-Butylcyclohexylacetat usw. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftsstoffalkohole sind beispielsweise 10-Undecen-1-ol, 2,6-Dimethylheptan-2-ol, 2-Methylbutanol, 2-Methylpentanol, 2-Phenoxyethanol, 2-Phenylpropanol usw. Duftstoffe bzw. Parfümöle können auch natürliche Riechstoffgemische sein, wie sie aus pflanzlichen Quellen zugänglich sind. Bei den Duftstoffen bzw. Parfümölen kann es sich auch um ätherische Öle, wie z.B. Angelikawurzelöl, Anisöl, Arnikablütenöl usw. handeln. Die Gesamtmenge des mindestens einen Duftstoffs in der erfindungsgemäßen Zubereitung, wie z.B. Wasch- oder Reinigungsmittel oder kosmetisches Mittel, beträgt vorzugsweise zwischen 0,001 und 5 Gew.-%, vorteilhafterweise zwischen 0,01 und 4 Gew.-%, weiter vorteilhaft zwischen 0,1 und 3 Gew.-% sowie ganz besonders bevorzugt zwischen 0,5 und 2 Gew.-% bezogen auf die Gesamtmenge der Zubereitung. Bevorzugt werden Mischungen verschiedener Duftstoffe (aus den verschiedenen oben genannten Duftstoffklassen) verwendet, die gemeinsam eine ansprechende Duftnote erzeugen. In diesem Fall entspricht die Gesamtmasse der Duftstoffmischung bezogen auf die Gesamtmasse der Zubereitung der Gesamtmenge des mindestens einen Duftstoffs in der erfindungsgemäßen Zubereitung.

Vorzugsweise handelt es sich bei der offenbarten Zubereitung um ein Textil- oder Oberflächenbehandlungsmittel, wobei die genannten Mittel überaus bevorzugt zusätzlich mindestens ein Tensid ausgewählt aus der Gruppe bestehend aus anionischen, kationischen, nichtionischen, zwitterionischen, amphoteren Tensiden oder deren Mischungen enthalten.

Vorzugsweise handelt es sich bei der offenbarten Zubereitung um ein Textilbehandlungsmittel in Form eines Waschmittels, Weichspülers, weichmachenden Waschmittels oder Waschhilfsmittels.

Vorzugsweise ist das offenbarte Keton der allgemeinen Formel (I) oder die Mischung verschiedener Ketone der allgemeinen Formel (I) in der Zubereitung in Form eines Waschmittels, Weichspülers, weichmachenden Waschmittels oder Waschhilfsmittels in Mengen von vorzugsweise zwischen 0,001 und 5 Gew.-%, vorteilhafterweise zwischen 0,01 und 4 Gew.-%, weiter vorteilhaft zwischen 0,1 und 3 Gew.-%, insbesondere zwischen 0,5 und 2 Gew.-%, jeweils bezogen auf die Gesamtmenge der Zubereitung, enthalten.

Eine Zusammensetzung enthaltend mindestens ein Keton der allgemeinen Formel (I), wobei die genannte Zusammensetzung ein Textil- oder Oberflächenbehandlungsmittel ist, vorzugsweise ein Waschmittel, Weichspüler, Waschhilfsmittel oder Reinigungsmittel ist, wobei das genannte Keton vorzugsweise in Mengen zwischen 0,001 und 5 Gew.-%, vorteilhafterweise zwischen 0,01 und 4 Gew.-%, weiter vorteilhaft zwischen 0,1 und 3 Gew.-%, insbesondere zwischen 0,5 und 2 Gew.-%, jeweils bezogen auf die Gesamtmenge der Zusammensetzung, enthalten ist, ist somit ein weiterer Gegenstand der Offenbarung.

Das Textilbehandlungsmittel kann dabei fest oder flüssig sein, wobei flüssige Waschmittel bevorzugt sind. Für den Fall, dass das offenbarte Mittel ein Waschmittel ist, ist es bevorzugt, dass es mindestens ein Tensid ausgewählt aus der Gruppe bestehend aus anionischen, nichtionischen, zwitterionischen und amphoteren Tensiden enthält. Für den Fall, dass das erfindungsgemäße Mittel ein weichmachendes Waschmittel ("2in1") ist, ist es bevorzugt, dass es eine weichmachende Komponente sowie mindestens ein Tensid ausgewählt aus der Gruppe bestehend aus anionischen, nichtionischen, zwitterionischen und amphoteren Tensiden enthält. Waschhilfsmittel werden zur gezielten Vorbehandlung der Wäsche vor dem Waschen bei Flecken oder starker Verschmutzung eingesetzt. Zu den Waschhilfsmitteln gehören beispielsweise Vorbehandlungsmittel, Einweichmittel, Entfärber und Fleckensalz.

Für den Fall, dass das offenbarte Mittel ein Weichspüler ist, ist es bevorzugt, dass es eine weichmachende Komponente enthält. Weichspüler sind als Mittel bevorzugt, da sie erst im letzten Schritt eines konventionellen Textilwaschvorgangs, dem Spülgang, in Kontakt mit den Textilien kommen und somit eine möglichst große Menge der Duftstoffe auf das Textil aufziehen kann, ohne dass die Gefahr besteht, dass die Duftstoffe bei anschließenden Schritten wieder entfernt werden. Es ist ganz besonders bevorzugt, dass die weichmachende Komponente eine alkylierte, quaternäre Ammoniumverbindung ist, wobei mindestens eine Alkylkette durch eine Ester- oder Amidogruppe unterbrochen ist. Die weichmachende Komponente umfasst beispielsweise quaternäre Ammoniumverbindungen wie Monoalk(en)yltrimethylammonium-Verbindungen, Dialk(en)yldimethylammonium-Verbindungen, Mono-, Di- oder Triester von Fettsäuren mit Alkanolaminen. Weitere erfindungsgemäß verwendbare weichmachende Komponenten stellen quaternisierten Proteinhydrolysate oder protonierte Amine dar. Weiterhin sind auch kationische Polymere geeignete weichmachende Komponente. Ebenfalls einsetzbar sind polyquaternierte Polymere (z.B. Luviquat® Care von BASF) und auch kationische Biopolymere auf Chitinbasis und deren Derivate, beispielsweise das unter der Handelsbezeichnung Chitosan® (Hersteller: Cognis) erhältliche Polymer. Weitere geeignete weichmachende Komponenten umfassen protonierte oder quaternierte Polyamine. Besonders bevorzugte weichmachende Komponenten sind alkylierte quaternäre Ammoniumverbindungen, von denen mindestens eine Alkylkette durch eine Estergruppe und/oder Amidogruppe unterbrochen ist. Ganz besonders bevorzugt sind N-Methyl-N-(2-hydroxyethyl)-N,N-(ditalgacyloxyethyl)ammonium-methosulfat oder Bis-(palmitoyloxyethyl)-hydroxyethyl-methyl-ammonium-methosulfat. Die offenbarte Zubereitung in Form von Weichspülern kann auch nichtionische weichmachende Komponenten enthalten, wie vor allem Polyoxyalkylenglycerolalkanoate, Polybutylene, langkettige Fettsäuren, ethoxylierte Fettsäureethanolamide, Alkylpolyglucoside, insbesondere Sorbitanmono,-di- und - triester, und Fettsäureester von Polycarbonsäuren. In dem offenbarten Weichspüler als Zubereitung ist vorteilhafterweise die weichmachende Komponente in Mengen von 0,1 bis 80 Gew.-%, üblicherweise 1 bis 40 Gew.-%, vorzugsweise 2 bis 20 Gew.-% und insbesondere 3 bis 15 Gew.-% und der mindestens eine Duftstoff oder die Mischung verschiedener Duftstoffe in Mengen von vorteilhafterweise 0,1 bis 20 Gew.-%, vorzugsweise 1 bis 13 Gew.-% und insbesondere 2 bis 8 Gew.-%, jeweils bezogen auf die Gesamtmenge des offenbarten Mittels, enthalten.

Als weitere Komponente kann die Zubereitung in Form eines Weichspülers gegebenenfalls ein oder mehrere nichtionische Tenside enthalten, wobei solche eingesetzt werden können, die üblicherweise auch in Waschmitteln verwendet werden.

Es ist weiterhin bevorzugt, dass die Zubereitung in Form eines Textil- oder Oberflächenbehandlungsmittels zusätzlich weitere vorteilhafte Inhaltsstoffe enthält, die dem Fachmann bekannt sind. So kann die offenbarte Zusammensetzung in Form eines Textilbehandlungsmittels zusätzlich zu den Tensiden und/oder weichmachenden Verbindungen weitere Inhaltsstoffe enthalten, die die anwendungstechnischen und/oder ästhetischen Eigenschaften des Textilbehandlungsmittels weiter verbessern. Im Rahmen der vorliegenden Erfindung enthalten bevorzugte Textilbehandlungsmittel zusätzlich einen oder mehrere Stoffe aus der Gruppe der Gerüststoffe, Bleichmittel, Bleichaktivatoren, Enzyme, Elektrolyte, nichtwässrigen Lösungsmittel, pH-Stellmittel, Parfüme, Parfümträger, Fluoreszenzmittel, Farbstoffe, Hydrotope, Schauminhibitoren, Silikonöle, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Konservierungsmittel, Korrosionsinhibitoren, Antistatika, Bittermittel, Bügelhilfsmittel, Phobier- und Imprägniermittel, Quell- und Schiebefestmittel, neutrale Füllsalze sowie UV-Absorber. Als Gerüststoffe, die in den Textilbehandlungsmitteln enthalten sein können, sind insbesondere Silikate, Aluminiumsilikate (insbesondere Zeolithe), Carbonate, Salze organischer Di- und Polycarbonsäuren sowie Mischungen dieser Stoffe zu nennen.

Die Herstellung der Weichspüler als Textilbehandlungsmittel kann nach dem Fachmann geläufigen Techniken zur Herstellung von Weichspülern erhalten werden. Dies kann beispielsweise durch Aufmischen der Rohstoffe, gegebenenfalls unter Einsatz von hochscherenden Mischapparaturen, geschehen. Es empfiehlt sich ein Aufschmelzen der weichmachenden Komponente(n) und ein nachfolgendes Dispergieren der Schmelze in einem Lösungsmittel, vorzugsweise Wasser. Die weiteren Inhaltsstoffe können durch einfaches Zumischen in die Weichspüler integriert werden. Die Herstellung der flüssigen Waschmittel als Textilbehandlungsmittel erfolgt mittels üblicher und bekannter Methoden und Verfahren in dem beispielsweise die Bestandteile einfach in Rührkesseln vermischt werden, wobei Wasser, nichtwässrige Lösungsmittel und Tenside, zweckmäßigerweise vorgelegt werden und die weiteren Bestandteile hinzugefügt werden. Ein gesondertes Erwärmen bei der Herstellung ist nicht erforderlich, wenn es gewünscht ist, sollte die Temperatur der Mischung 80°C nicht übersteigen.

Als Oberflächenbehandlungsmittel kann die offenbarte Zubereitung zur Reinigung harter Oberflächen eingesetzt werden. Es kann sich dabei beispielsweise um Geschirrreinigungsmittel handeln, die zur manuellen oder maschinellen Reinigung von Geschirr eingesetzt werden. Es kann sich auch um übliche Industrie- oder Haushaltsreiniger handeln, mit denen harte Oberflächen wie Möbeloberflächen, Fliesen, Kacheln, Wand- und Bodenbeläge gereinigt werden. Als harte Oberflächen kommen neben Geschirr auch alle übrigen harten Oberflächen, insbesondere aus Glas, Keramik, Kunststoff oder Metall, in Haushalt und Gewerbe in Frage. Dabei kann es sich bei den Textil- oder Oberflächenbehandlungsmitteln um feste oder flüssige Formulierungen handeln, wobei feste Formulierungen als Pulver, Granulat, Extrudat, in Tab-Form, als Tablette oder als gepresster und/oder geschmolzener Formkörper vorliegen können. Bei flüssigen Formulierungen kann es sich um Lösungen, Emulsionen, Dispersionen Suspensionen, Mikroemulsionen, Gels oder Pasten handeln. Als Oberflächenbehandlungsmittel kann das Mittel dementsprechend übliche Inhaltsstoffe von Reinigungsmitteln in üblichen Mengen enthalten.

In einer weiteren bevorzugten Ausführungsform der Offenbarung handelt es sich bei der Zubereitung um ein kosmetisches Mittel zur Haut- und Haarbehandlung. Vorzugsweise ist das offenbarte Keton der allgemeinen Formel (I) oder die Mischung verschiedener offenbarten Ketone der allgemeinen Formel (I) in der Zubereitung in Form eines kosmetischen Mittels zur Haut- und Haarbehandlung in Mengen von vorzugsweise zwischen 0,001 und 5 Gew.-%, vorteilhafterweise zwischen 0,01 und 4 Gew.-%, weiter vorteilhaft zwischen 0,1 und 3 Gew.-%, insbesondere zwischen 0,5 und 2 Gew.-%, jeweils bezogen auf die Gesamtmenge der Zubereitung, enthalten. Eine Zusammensetzung enthaltend mindestens ein offenbarten Keton der allgemeinen Formel (I), wobei die genannte Zusammensetzung ein kosmetisches Mittel zur Haut- und Haarbehandlung ist, wobei das genannte Keton vorzugsweise in Mengen zwischen 0,001 und 5 Gew.-%, vorteilhafterweise zwischen 0,01 und 4 Gew.-%, weiter vorteilhaft zwischen 0,1 und 3 Gew.-%, insbesondere zwischen 0,5 und 2 Gew.-%, jeweils bezogen auf die Gesamtmenge der Zusammensetzung, enthalten ist, ist ein weiterer Gegenstand der vorliegenden Offenbarung.

Gegenstand der vorliegenden Erfindung ist das bereits genannte Verfahren zur Herstellung eines erfindungsgemäßen Ketons der allgemeinen Formel (I) umfassend folgende Schritte:
a) Hydroborierung einer Verbindung der allgemeinen Formel (III) 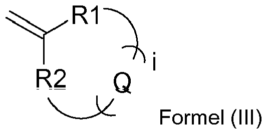 wobei i = 1 oder 2 ist und R1 und R2 unabhängig voneinander für ein sekundäres, tertiäres oder quartäres C-Atom stehen und Q für mindestens eine R1 und R2 verbrückende zweiwertige substituierte oder unsubstituierte Gruppe mit 1 bis 10 Kohlenstoffatomen steht,
b) Umsetzung des in Schritt a) erzeugten Organoboraddukts mit einer Verbindung der allgemeinen Formel (IV), 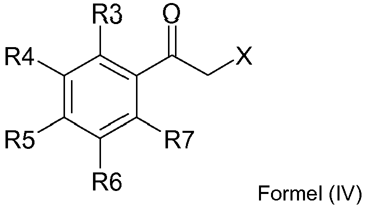 wobei X ein Halogenatom ist und R3, R4, R5, R6 und R7 unabhängig voneinander für Wasserstoff, ein Halogenatom, NO2, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

In einer besonders bevorzugten Ausführungsform des genannten Verfahrens steht X in einer Verbindung der allgemeinen Formel (IV) für -Cl oder -Br, insbesondere für -Br. In einer ebenfalls bevorzugten Ausführungsform des genannten Verfahrens ist eine Verbindung der allgemeinen Formel (IV) bevorzugt, in der vier der fünf Arylsubstituenten R3, R4, R5, R6 und R7 für Wasserstoff stehen. Vorzugsweise stehen R3, R4, R6 und R7 für Wasserstoff, während der Substituent in para-Position R5 für ein Halogenatom, NO2, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen steht. In einer überaus bevorzugten Ausführungsform der Erfindung steht R5 für -Cl, -Br, -NO2 oder eine Alkyl- oder Alkoxygruppe umfassend 1 bis 4 C-Atome. Vorzugsweise handelt es sich bei der linearen oder verzweigten, substituierten oder unsubstituierten Alkylgruppe um eine Methyl- oder Ethylgruppe und/oder bei der linearen oder verzweigten, substituierten oder unsubstituierten Alkoxygruppe um eine Methoxy-, Ethoxy-, Isopropoxy- oder tert-Butoxy-Gruppe. Unter dem Begriff Hydroborierung ist im Sinne der Erfindung eine 1,2-Addition eines Organoboranreagenzes an zumindest eine semicyclische Doppelbindung der Verbindung der allgemeinen Formel (III) zu verstehen, wodurch zwischen dem exocyclischen Cβ-Atom der semicyclischen Doppelbindung und dem Bor-Atom des verwendeten Organoboranreagenzes eine kovalente Kohlenstoff-Bor-Bindung und damit ein Organoboraddukt gebildet wird. Geeignete Verfahren zur Hydroborierung sind in J. March, Advanced Organic Chemistry, 4. Aufl., S. 783-789 beschrieben, worauf hier Bezug genommen wird.

Vorzugsweise führt man die Hydroborierung in einem Lösungsmittel durch. Geeignete Lösungsmittel für die Hydroborierung sind beispielsweise acyclische Ether wie Diethylether, Methyl-tert.-butylether, Dimethoxyethan, Diethylenglykoldimethylether, Triethylenglykoldimethylether, cyclische Ether wie Tetrahydrofuran oder Dioxan sowie Kohlenwasserstoffe wie Hexan oder Toluol oder Gemische davon. Die Reaktionstemperatur wird in der Regel von der Reaktivität des Hydroborierungsmittels bestimmt und liegt vorzugsweise zwischen dem Schmelz -und Siedepunkt des Reaktionsgemisches. Insbesondere kann die Reaktion in THF und/oder Diethylether bei Temperaturen zwischen -100°C und 40°C, vorzugsweise zwischen -40°C und 30°C und insbesondere zwischen 0°C und 20°C durchgeführt werden. Geeignete Organoboranreagenzien sind vorzugsweise sterisch anspruchsvolle Organoboranreagenzien, wobei diese beispielsweise ausgewählt werden können aus der Gruppe bestehend aus 9-Borabicyclo[3.3.1]nonan (9-BBN), 1,1,2-Trimethylpropyl-boran (Thexylboran), Catecholboran oder Diisopinocampheylboran (Ipc2BH). Vorzugsweise setzt man das Organoboranreagenz äquimolar oder im Überschuß bezogen auf die mindestens eine semicyclische Doppelbindung der Verbindung der allgemeinen Formel (III) ein. Ein Vorteil des genannten Verfahrens liegt in der Tatsache, dass die gebildeten Organoboraddukte vorzugsweise nicht isoliert werden müssen, sondern ohne vorhergehende Aufreinigung direkt mit einer Verbindung der allgemeinen Formel (IV) umgesetzt werden können. Die genannte Umsetzung des Organoboranaddukts mit der Verbindung der allgemeinen Formel (IV) erfolgt vorzugsweise in einem geeigneten Lösungsmittel, beispielsweise in acyclischen Ethern wie Diethylether, Methyl-tert.-butylether, Dimethoxyethan, Diethylenglykoldimethylether, Triethylenglykoldimethylether, in cyclischen Ethern wie Tetrahydrofuran oder in Dioxan sowie in Kohlenwasserstoffen wie Hexan oder Toluol oder in Gemischen davon, wobei die beschriebene Umsetzung überaus bevorzugt in Gegenwart eines Alkalimetallalkoholats durchgeführt wird. Die Reaktionstemperatur wird in der Regel von der Reaktivität der Verbindung der allgemeinen Formel (IV) und/oder von der Reaktivität des Organoboraddukts bestimmt und liegt vorzugsweise zwischen dem Schmelz -und Siedepunkt des Reaktionsgemisches. Insbesondere kann die Reaktion in THF und/oder Diethylether bei Temperaturen zwischen -100°C und 40°C, vorzugsweise zwischen -90°C und 0°C und insbesondere zwischen -78°C und -40°C durchgeführt werden.

In einer überaus bevorzugten Ausführungsform wird das genannte Alkalimetallalkoholat ausgewählt aus Kalium-methanolat, Natrium-methanolat, Kalium-ethanolat, Natrium-ethanolat, Kaliumpropylat, Natrium-propylat, Kalium-tert-butanolat, Natrium-tert-butanolat, Kalium-2,6-di-tert-butylphenolat, Natrium-2,6-di-tert-butylphenolat und aus deren beliebigen Mischungen. Vorzugsweise setzt man das Alkalimetallalkoholat äquimolar oder im leichten Überschuss (1,05 bis 1,30 eq.) bezogen auf das gebildete Organoboranaddukt ein.

Die Aufreinigung des gebildeten Ketons der allgemeinen Formel (I) erfolgt vorzugsweise durch Kristallisation, Destillation und/oder Säulenchromatographie. Ein Vorteil des beschriebenen Herstellungsverfahrens liegt in der Tatsache, dass die Ketone der allgemeinen Formel (I) in der Regel in einer einstufigen konvergenten Synthese ohne aufwendige Schutzgruppenoperationen direkt und kostengünstig hergestellt werden können. Es entsteht somit ein Duftspeicherstoff AB, der im Wesentlichen aus einem Photorezeptor (A) und einem cyclischen Alken mit semicyclischer Doppelbindung (B) zusammengesetzt ist. Durch Bestrahlung zerfällt der genannte Duftspeicherstoff AB dann wieder problemlos in seine einzelnen Bestandteile A und B.

### Ausführungsbeispiele

### Beispiel 1: Darstellung eines Ketons der allg. Formel (I)

Synthese von 3-(6, 6-Dimethylbicyclo [3.1.1]-heptan-2-yl)-1-phenylpropan-1-on 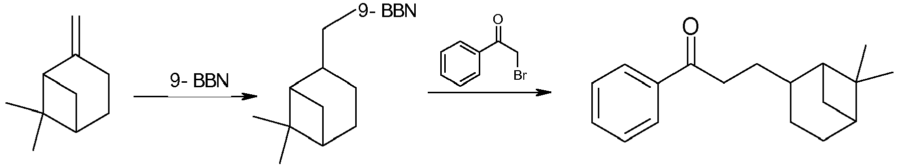 1.36 g (-)-β-Pinen (10 mmol) wurden in 5 mL THF gelöst. Zu dieser Lösung wurden 21 mL einer 0.5 molaren Lösung von 9-BBN in THF (10.5 mmol) bei 0°C zugefügt. Die Reaktionsmischung wurde langsam auf Raumtemperatur gebracht und weitere 3 Stunden gerührt. Die entstandene Lösung wurde auf -78°C abgekühlt und anschließend eine Lösung von 1.12 g Kalium-tert-butanolat (10 mmol) in 10 mL THF zu der abgekühlten Reaktionslösung zugegeben. Nach kurzer Zeit wurden 2 g α-Bromacetophenon (10 mmol) portionsweise bei konstantem Rühren hinzu gegeben. Die Reaktionsmischung wurde langsam auf Raumtemperatur gebracht und 4 Stunden bei Raumtemperatur gerührt. Das entstandene Produktgemisch wurde mit 50 mL n-Pentan versetzt, 3-mal mit jeweils 10 mL einer 3 N Natriumhydroxidlösung und mit 10 ml Wasser gewaschen. Die organische Phase wurde über Magnesiumsulfat getrocknet. Das Filtrat wurde bei vermindertem Druck von Lösungsmittel befreit. Das Rohprodukt wurde säulenchromatographisch gereinigt. Es wurde 140 mg (10 %) des oben genannten Produkts erhalten.

### Analytische Daten:

### Rf = 0.75 (Petrolether:Ether = 9:1)

¹H NMR (300 MHz, CDCl₃) (δ, ppm): 1.03 (s, 3H), 1.19 (s, 3H), 1.76-2.10 (m, 8H), 2.34 (q, 1H, 7.2 Hz), 2.95 (t, 2H, 8.1 Hz), 7.46 (t, 2H, 7.8 Hz,), 7.55 (t, 1H, 7.2 Hz), 7.96 (d, 2H, 8.1 Hz); ¹³C NMR (75 MHz, CDCl₃) (δ, ppm): 22.4, 23.3, 26.5, 28.2, 32.0, 33.8, 37.3, 38.7, 41.3, 41.5, 46.2, 128.1, 128.5, 132.8, 137.1, 200.8;
GC/MS (50-300M), m/z: 256, 238, 223, 195, 133, 119, 105, 91, 77, 55, 41.

### Beispiel 2 : Belichtung von 3-(6, 6-Dimethylbicyclo [3.1.1]-heptan-2-yl)-1-phenylpropan-1-on

20 mg 3-(6,6-Dimethylbicyclo[3.1.1]heptan-2-yl)-1-phenylpropan-1-on wurden in 8 ml Methanol gelöst. Die Reaktionslösung wurde in einem Multilamp-Photoreactor (8 W-Lampen (4×), Firma Luxchem) mit Emissionmaximum λ = 350 nm eine Stunde lang belichtet. Die Reaktion wurde mit Hilfe der GC/MS-Spektrometrie verfolgt. Nach spätestens 60 Minuten Belichtung wurde die weitgehend vollständige Umwandlung von 3-(6,6-Dimethylbicyclo[3.1.1]heptan-2-yl)-1-phenylpropan-1-on in Acetophenon und β-Pinen beobachtet.

### Beispiel 3: Riechtest

### Für den im Folgenden beschriebenen Riechtest wurden 0,2 mmol 3-(6,6-Dimethylbicyclo[3.1.1]heptan-2-yl)-1-phenylpropan-1-on (Photocage Pinen) in 1 ml Acteon gelöst. In die Lösung wurde ein Riechstreifen 2 cm tief eingetaucht, der anschließend unter Lichtausschluss bei 20°C getrocknet wurde.

Zum Vergleich wurden Lösungen von 0,1 mmol beta-Pinen und 0.1 mmol Acetophenon in je 1 ml Aceton und eine Mischung von 0.1 mmol beta-Pinen und 0.1 mmol Acetophenon in 1 ml Aceton hergestellt. Anschließend wurde in jede Lösung ein Riechstreifen 2 cm tief eingetaucht, wobei jeder Riechstreifen anschließend unter Lichtausschluss bei 20°C getrocknet wurde.
Nach erfolgreicher Trocknung wird jeder Riechstreifen über den gesamten Testzeitraum hinweg mit einer handelsüblichen Leuchtstoffröhre (gemäß DIN 5035 neutralweiß (nw); Farbtemperatur 3300 bis 5500 K) bestrahlt und die Duftintensität zu den jeweils angegebenen Zeitpunkten bestimmt.

Die Duftintensität wurde von 3 trainierten Probanden auf einer Skala von 0 bis 6 bewertet, wobei 6 die höchste Note ist und 0 für keine Duftwahrnehmung steht.

Definition der Skalierung:
- 6: unangenehm stark
- 5: sehr stark
- 4: stark
- 3: intensiv
- 2: angenehm
- 1: wahrnehmbar
- 0: nicht mehr wahrnehmbar

**Die Ergebnisse der Riechtests sind in der folgenden Tabelle dargestellt, wobei die angegebenen Werte den Bereich der Duftwahrnehmung der Probandengruppe widerspiegeln.**

| | nach 1 Minute | nach 20 Minuten | nach 45 Minuten | nach 1,5 Stunden | nach 24 Stunden |
|---|---|---|---|---|---|
| Photocage Pinen | chemischer Geruch stark nach Acetophenon 1 | 0-1 | 0-1 | 0 wird durch Tageslichlampe erneut riechbar 0-1 | 0 wird durch Tageslichlampe erneut riechbar 0-1 |
| Acetophenon | 3 | 3 | 2-3 | 2 | 0 |
| beta-Pinen | 0-1 | 0 | 0 | 0 | 0 |
| Acetophenon/ beta-Pinen | nur Acetophenon wahrnehmbar 2-3 | 2-3 | 2 | 2 | 0 |

Es zeigt sich, dass die ca. 1-minütige Bestrahlung mit einer 20 Watt Tageslichtlampe (LifeLite Full Spectrum Daylight Lamp) auch noch nach 24 Stunden die photoinduzierte Freisetzung des Geruchsstoffs Pinen bewirkt.

## Patentansprüche

1. Verfahren zur lang anhaltenden Beduftung von Oberflächen, **dadurch gekennzeichnet, dass** ein Keton auf die zu beduftende Oberfläche aufgebracht wird und die genannte Oberfläche anschließend einer elektromagnetischen Strahlung umfassend die Wellenlängen von 200 bis 400 nm ausgesetzt wird, wobei das Keton der allgemeinen Formel (I) genügt, 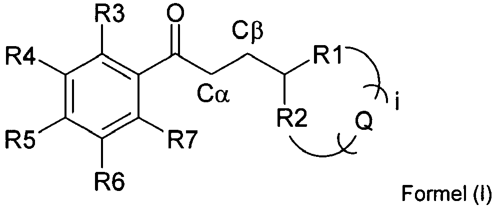 wobei
i =1 oder 2 ist,
R3, R4, R5, R6 und R7 unabhängig voneinander für Wasserstoff, ein Halogenatom, NO₂, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen und
R1 und R2 unabhängig voneinander für ein sekundäres, tertiäres oder quartäres C-Atom stehen und
Q für mindestens eine R1 und R2 verbrückende zweiwertige substituierte oder unsubstituierte Gruppe mit 1 bis 10 Kohlenstoffatomen steht,
wobei durch Spaltung der Cα-Cβ-Bindung ein Keton der allgemeinen Formel (II) 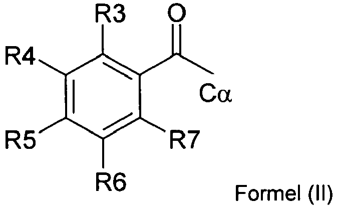 und eine Verbindung der allgemeinen Formel (III) gebildet wird, 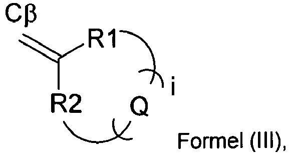 wobei es sich bei der Verbindung der allgemeinen Formel (III) um ein cyclisches Terpen oder cyclisches Terpenoid mit einer semicyclischen Doppelbindung handelt,
ausgewählt aus der Gruppe bestehend aus β-Phellandren (p-Mentha-1(7),2-dien), α-Fenchen (7,7-Dimethyl-2-methylen-bicyclo[2.2.1]heptan), β-Fenchen (2,2-Dimethyl-5-methylenbicyclo-[2.2.1]heptan), Camphen (2,2-Dimethyl-3-methylenbicyclo[2.2.1]heptan), Sabinen (4-Methylen-1-(1-methylethyl)bicyclo-[3.1.0]hexan), Pinocarveol, β-Pinen (6,6-Dimethyl-2-methylenbicyclo-[3.1.1]heptan), Trichodien (1,4-Dimethyl-4-(1-methyl-2-methylencyclopentyl)cyclohexen), β-Humulen ((1E,5E)-1,4,4 trimethyl-8-methylenecycloundeca-1,5-dien) oder γ-Humulen ((1E,6E)-1,8,8-trimethyl-5-methylenecycloundeca-1,6-dien) sowie aus deren Enantiomeren und/oder Diastereomeren.

2. Verfahren gemäß Anspruch 1 **dadurch gekennzeichnet, dass** bei dem Keton der allgemeinen Formel (I) vier der fünf Arylsubstituenten R3, R4, R5, R6 und R7 für Wasserstoff stehen.

3. Verfahren gemäß Anspruch 1 oder 2, **dadurch gekennzeichnet, dass** bei dem Keton der allgemeinen Formel (I) R3, R4, R5, R6 und R7 für Wasserstoff stehen.

4. Verfahren zur Herstellung eines Ketons der allgemeinen Formel (I) gemäß Anspruch 1 umfassend folgende Schritte:
a) Hydroborierung einer Verbindung der allgemeinen Formel (III) 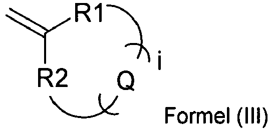 wobei i = 1 oder 2 ist und R1 und R2 unabhängig voneinander für ein sekundäres, tertiäres oder quartäres C-Atom stehen und Q für mindestens eine R1 und R2 verbrückende zweiwertige substituierte oder unsubstituierte Gruppe mit 1 bis 10 Kohlenstoffatomen steht,
b) Umsetzung des in Schritt a) erzeugten Organoboraddukts mit einer Verbindung der allgemeinen Formel (IV), 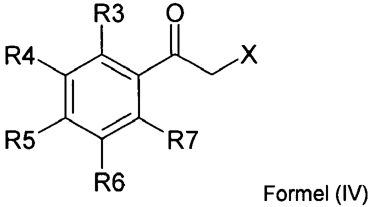 wobei X ein Halogenatom ist und R3, R4, R5, R6 und R7 unabhängig voneinander für Wasserstoff, ein Halogenatom, NO2, eine lineare oder verzweigte, substituierte oder unsubstituierte Alkoxygruppe mit 1 bis 15 C-Atomen oder eine lineare oder verzweigte, substituierte oder unsubstituierte Alkylgruppe mit 1 bis 15 C-Atomen stehen.

## Claims

1. A method for long-lasting fragrancing of surfaces, **characterized in that** a ketone is applied to the surface to be fragranced and said surface is then exposed to electromagnetic radiation having wavelengths of from 200 to 400 nm, wherein the ketone satisfies the general formula (I), 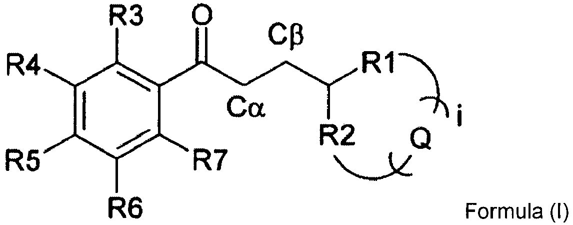 wherein
i = 1 or 2,
R3, R4, R5, R6 and R7, independently of one another, represent hydrogen, a halogen atom, NO₂, a linear or branched, substituted or unsubstituted alkoxy group having from 1 to 15 carbon atoms or a linear or branched, substituted or unsubstituted alkyl group having from 1 to 15 carbon atoms, and
R1 and R2, independently of one another, represent a secondary, tertiary or quaternary carbon atom, and
Q represents at least one R1 and R2 bridging, divalent, substituted or unsubstituted group having from 1 to 10 carbon atoms,
wherein a ketone of the general formula (II) 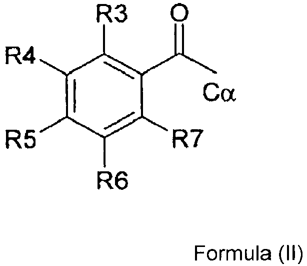 and a compound of the general formula (III) 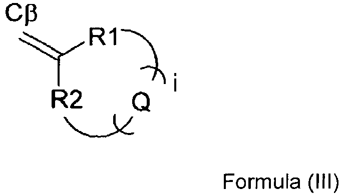 is formed by cleavage of the Cα-Cβ bond,
wherein the compound of the general formula (III) is a cyclic terpene or cyclic terpenoid having a semi-cyclic double bond, selected from the group consisting of β-phellandrene (p-mentha-1(7),2-diene), α-fenchene (7,7-dimethyl-2-methylenebicyclo[2.2.1]heptane), β-fenchene (2,2-dimethyl-5-methylenebicyclo[2.2.1]heptane), camphene (2,2-dimethyl-3-methylenebicyclo[2.2.1]heptane), sabinene (4-methylene-1-(1-methylethyl)bicyclo[3.1.0]hexane), pinocarveol, β-pinene (6,6-dimethyl-2-methylenebicyclo[3.1.1]heptane), trichodiene (1,4-dimethyl-4-(1-methyl-2-methylenecyclopentyl)cyclohexene), β-humulene ((1E,5E)-1,4,4 trimethyl-8-methylenecycloundeca-1,5-diene) or γ-humulene ((1E,6E)-1,8,8-trimethyl-5-methylenecycloundeca-1,6-diene) and enantiomers and/or diastereomers thereof.

2. The method according to claim 1, **characterized in that** in the ketone of the general formula (I), four of the five aryl substituents R3, R4, R5, R6 and R7 represent hydrogen.

3. The method according to either claim 1 or claim 2, **characterized in that** in the ketone of the general formula (I), R3, R4, R5, R6 and R7 represent hydrogen.

4. A method for producing a ketone of the general formula (I) according to claim 1, comprising the following steps:
a) hydroboration of a compound of the general formula (III) 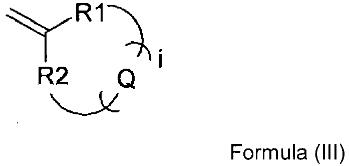 wherein i = 1 or 2 and R1 and R2, independently of one another, represent a secondary, tertiary or quaternary carbon atom and Q represents at least one R1 and R2 bridging, divalent, substituted or unsubstituted group having from 1 to 10 carbon atoms,
b) reacting the organoborane adduct that was produced in step a) with a compound of the general formula (IV), 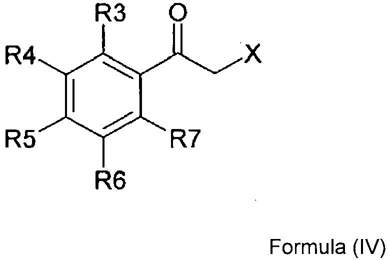 wherein X is a halogen atom and R3, R4, R5, R6 and R7, independently of one another, represent hydrogen, a halogen atom, NO₂, a linear or branched, substituted or unsubstituted alkoxy group having from 1 to 15 carbon atoms or a linear or branched, substituted or unsubstituted alkyl group having from 1 to 15 carbon atoms.

## Revendications

1. Procédé pour parfumer des surfaces sur une longue durée, **caractérisé en ce qu'**une cétone est appliquée sur la surface à parfumer et ladite surface est ensuite soumise à un rayonnement électromagnétique comprenant les longueurs d'ondes allant de 200 400 nm, la cétone ayant la formule générale (I) 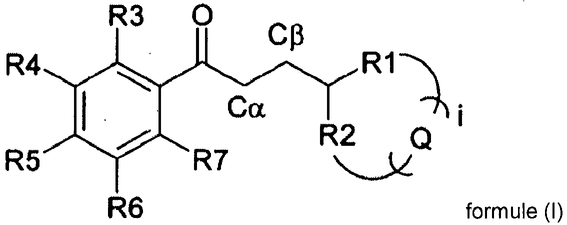 dans laquelle
i = 1 ou 2,
R3, R4, R5, R6 et R7 représentent indépendamment un atome d'hydrogène, un atome d'halogène, NO₂, un groupe alcoxy linéaire ou ramifié, substitué ou non substitué, ayant de 1 à 15 atomes de carbone, ou un groupe alkyle linéaire ou ramifié, substitué ou non substitué, ayant de 1 à 15 atomes de carbone et
R1 et R2 représentent indépendamment un atome de carbone secondaire, tertiaire ou quaternaire et
Q représente au moins un groupe divalent, substitué ou non substitué, pontant R1 et R2, ayant de 1 à 10 atomes de carbone,
un clivage de la liaison Cα-Cβ permettant de former une cétone de la formule générale (II) 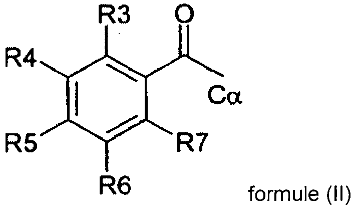 et un composé de la formule générale (III) 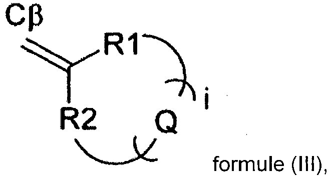 le composé de la formule générale (III) étant un terpène cyclique ou un terpénoïde cyclique ayant une double liaison semi-cyclique, choisi dans le groupe constitué par le ß-phellandrène (p-mentha-1(7),2-diène), le α-fenchène (7,7-diméthyl-2-méthylène-bicyclo-[2.2.1]heptane), le β-fenchène (2,2-diméthyl-5-méthylène-bicyclo-[2.2.1]heptane), le camphène (2,2-diméthyl-3-méthylène-bicyclo-[2.2.1] heptane), le sabinène (4-méthylène-1-(1-méthyléthyl)-bicyclo-[3.1.0]hexane), le pinocarvéol, le ß-pinène (6,6-diméthyl-2-méthylène-bicyclo-[3.1.1]heptane), le trichodiène (1,4-diméthyl-4-(1-méthyl-2-methylène-cyclopentyl)cyclohexène), le ß-humulène ((1E,5E)1,4,4-triméthyl-méthylène-cycloundéca-1,5-diène) ou le γ-humulène ((1E,6E)-1,8,8-triméthyl-5-méthylène-cycloundéca-1,6-diène), ainsi que leurs énantiomères et/ou diastéréomères.

2. Procédé selon la revendication 1, **caractérisé en ce que**, dans la cétone de la formule générale (I), quatre des cinq substituants aryle R3, R4, R5, R6 et R7 représentent un atome d'hydrogène.

3. Procédé selon la revendication 1 ou 2, **caractérisé en ce que**, dans la cétone de la formule générale (I), R3, R4, R5, R6 et R7 représentent un atome d'hydrogène.

4. Procédé de préparation d'une cétone de la formule générale (I) selon la revendication 1, comprenant les étapes consistant à :
a) effectuer l'hydroboration d'un composé de la formule générale (III) 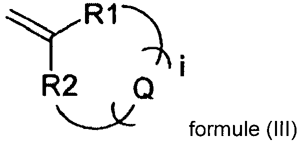 dans laquelle i = 1 ou 2 et R1 et R2 représentent indépendamment un atome de carbone secondaire, tertiaire ou quaternaire et Q représente au moins un groupe divalent, substitué ou non substitué, pontant R1 et R2, ayant 1 à 10 atomes de carbone,
b) faire réagir le produit d'addition organobore généré à l'étape a) avec un composé de la formule générale (IV), 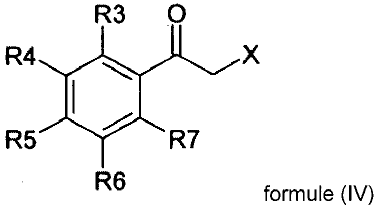 dans laquelle X est un atome d'halogène et R3, R4, R5, R6 et R7 représentent indépendamment l'hydrogène, un atome d'halogène, NO₂, un groupe alcoxy linéaire ou ramifié, substitué ou non substitué, ayant de 1 à 15 atomes de carbone, ou un groupe alkyle linéaire ou ramifié, substitué ou non substitué, ayant de 1 à 15 atomes de carbone.
